# EUROPÄISCHE PATENTSCHRIFT

(11) **EP 4 291 869 B1**
(45) Veröffentlichungstag und Bekanntmachung des Hinweises auf die Patenterteilung: **29.07.2026**
(21) Anmeldenummer: 22713536.5
(22) Anmeldetag: 10.02.2022
(51) Int. Cl.: G01N 15/06, G01N 33/28

(54) **VERFAHREN ZUR ÜBERWACHUNG EINES GETRIEBES IN EINEM LUFTFAHRZEUG, ÜBERWACHUNGSSYSTEM FÜR EIN GETRIEBE IN EINEM LUFTFAHRZEUG SOWIE LUFTFAHRZEUG MIT DEM ÜBERWACHUNGSSYSTEM**
METHOD FOR MONITORING A TRANSMISSION IN AN AIRCRAFT, MONITORING SYSTEM FOR A TRANSMISSION IN AN AIRCRAFT AND AIRCRAFT WITH THE MONITORING SYSTEM
PROCEDE DE SURVEILLANCE D'UNE TRANSMISSION DANS UN VEHICULE AERIEN, SYSTEME DE SURVEILLANCE D'UNE TRANSMISSION DANS UN VEHICULE AERIEN ET VEHICULE AERIEN AVEC LE SYSTEME DE SURVEILLANCE

(30) Priorität: 11.02.2021 DE 102021201290
(43) Veröffentlichungstag der Anmeldung: 20.12.2023
(73) Patentinhaber: Airbus Helicopters Technik GmbH, 34379 Calden (DE)
(72) Erfinder: LIEDER, Sergej, 34123 Kassel (DE); RÖSENER, Timm, 34260 Kaufungen (DE); LITZBA, Jörg, 34233 Fuldatal (DE)
(74) Vertreter: GPI Brevets
(86) Internationale Anmeldenummer: PCT/EP2022/053184
(87) Internationale Veröffentlichungsnummer: WO 2022/171710

(56) Entgegenhaltungen:
- EP-A2- 2 014 877
- DE-A1- 102012 219 242
- US-A- 4 598 280
- US-A- 5 179 346

## Beschreibung

Die Erfindung betrifft ein Verfahren zur Überwachung eines Getriebes in einem Luftfahrzeug mit den Merkmalen des Oberbegriffs des Anspruchs 1. Ferner betrifft die Erfindung ein Überwachungssystem für ein Getriebe in einem Luftfahrzeug sowie ein Luftfahrzeug mit dem Überwachungssystem.

Bei Getrieben in Flugzeugen oder Helikoptern können hohe Momente auftreten, welche einen Verschleiß oder einen natürlichen Abrieb an den Getriebekomponenten verursachen können. Es ist daher notwendig den Zustand des Getriebes zu überwachen, um einen einwandfreien und sicheren Flugbetrieb zu gewährleisten. Hierzu sind Sensoren zur Detektion von ferromagnetischen Partikeln bekannt, bei denen ein magnetisches Feld erzeugt wird, um die ferromagnetischen Partikeln anzuziehen und mittels eines geeigneten Detektors zu detektieren.

Beispielsweise offenbart die Druckschrift DE 102012219242 A1 eine Messvorrichtung zur Detektierung ferromagnetischer Partikel, die beweglich in einem Flüssigkeitsvolumen angeordnet sind, wobei die Messvorrichtung ein Magnetmittel zur Erzeugung eines magnetischen Feldes in dem Flüssigkeitsvolumen, ein Messmittel für den elektrischen Gesamtwiderstand des Flüssigkeitsvolumens mit den darin befindlichen ferromagnetischen Partikeln sowie ein Mittel zur Funktionsprüfung der Messvorrichtung mit mindestens einem niederohmigen Widerstand zwischen 50 und 500 Ohm zur Durchführung einer Widerstandsmessung aufweist.

US 4,598,280 A offenbart sowohl ein Verfahren als auch ein Überwachungssystem zur Überwachung eines Getriebes in einem Luftfahrzeug.

Der Erfindung liegt die Aufgabe zugrunde, ein Verfahren zur Überwachung eines Getriebes vorzuschlagen, welches eine qualitative Aussage zum Zustand des Getriebes liefert. Ferner ist es eine Aufgabe der Erfindung ein entsprechendes Überwachungssystem sowie ein Luftfahrzeug mit dem Überwachungssystem vorzuschlagen.

Diese Aufgabe wird durch ein Verfahren mit den Merkmalen des Anspruchs 1, ein Überwachungssystem mit den Merkmalen des Anspruchs 6 sowie ein Luftfahrzeug mit den Merkmalen des Anspruchs 11 gelöst. Bevorzugte oder vorteilhafte Ausführungsformen der Erfindung ergeben sich aus den Unteransprüchen, der nachfolgenden Beschreibung sowie den beigefügten Figuren.

Im Rahmen der Erfindung wird ein Verfahren zur Überwachung eines Getriebes in einem Luftfahrzeug vorgeschlagen. Das Getriebe weist einen Getriebeabschnitt mit mindestens oder genau einem Messbereich auf. Der Getriebeabschnitt kann dabei durch einen Getriebeinnenraum des Getriebes oder einen Teilraum des Getriebeinnenraums definiert sein. Bevorzugt ist der Messbereich in einem Bereich eines Ölsumpfs und/oder eines Bodenbereichs des Getriebes angeordnet. Alternativ kann der mindestens eine Messbereich jedoch auch an jeder beliebigen anderen Stelle des Getriebeabschnitts angeordnet sein, in welchem ein erhöhter Verschleiß oder Abrieb der Getriebekomponenten zu erwarten ist. Letztlich ist es vorgesehen, dass der Messbereich auf jeden Fall in einem Bereich angeordnet ist, in dem ein Ölfluss erfolgt. Beispielsweise kann durch eine Öldüse ein Ölvolumen in ein Lager eingespritzt werden, so dass das Öl beim Durchfließen des Lagers Partikel aufnimmt, welche dann durch den weiteren Ölfluss einem Partikelerkennungssensor zugeführt werden.

In dem Messbereich ist ein erster und ein zweiter elektrischer Kontakt angeordnet, wobei die beiden elektrischen Kontakte mit einem Abstand zueinander beabstandet angeordnet sind. Bevorzugt ist durch den Abstand ein Spalt zwischen den beiden Kontakten gebildet, durch welchen die Kontakte elektrisch voneinander getrennt sind. Insbesondere sind die beiden elektrischen Kontakte mit einer Stromquelle verbunden, wobei der Stromkreis in dem Messbereich unterbrochen ist. Die beiden elektrischen Kontakte können mit einem Abstand von weniger als 1 mm, vorzugsweise weniger als 0,5 mm, im Speziellen weniger als 0,1 mm zueinander angeordnet sein. Alternativ oder optional ergänzend können durch die elektrischen Kontakte einzelne Partikel mit einer größer von mehr als 0,1 mm, vorzugsweise mehr als 0,5 mm, im Speziellen mehr als 1 mm detektiert werden. Dabei ist letztlich allerdings nicht die reine Partikelgröße ausschlaggebend für eine erfolgreiche Detektion, sondern vielmehr das von den Partikeln gebildete Volumen, welches einen elektrischen Widerstand vorgibt und überhaupt erst eine Stromleitung zwischen den Kontakten ermöglicht.

Des Verfahren umfasst folgende Schritte:
- Erzeugen eines Magnetfelds in dem Getriebeabschnitt, wobei das Magnetfeld dazu eingerichtet ist, mindestens einen in dem Getriebeabschnitt angeordneten ferromagnetischen, insbesondere elektrisch leitfähigen, Partikel zum Messbereich hin zu bewegen, sodass sich eine, insbesondere elektrische leitfähige, Brücke aus ein oder mehreren der ferromagnetischen Partikeln zwischen den beiden elektrischen Kontakten bildet oder gebildet werden;
- Erzeugen eines Messwertes basierend auf einer elektrischen Kenngröße zwischen den beiden elektrischen Kontakten;
- Ermitteln eines Maß für die Kritikalität eines Flugbetriebs basierend auf dem Messwert.

Das Magnetfeld kann beispielsweise permanent oder temporär, insbesondere für die Dauer der Messung, erzeugt werden. Bevorzugt wird das Magnetfeld innerhalb des Spalts erzeugt. Insbesondere ist ein Wirkbereich des Magnetfeldes, also der Bereich in welchem die ferromagnetischen Partikel von dem Magnetfeld angezogen werden, innerhalb des Messbereichs bzw. überlappend zu dem Messbereich angeordnet.

Insbesondere wird die Brücke nur dann gebildet, wenn ein oder mehrere der ferromagnetischen Partikel in dem Messbereich angeordnet sind. Vorzugsweise wird bei der Bildung der elektrisch leitfähigen Brücke der Abstand bzw. Spalt zwischen den beiden elektrischen Kontakten überbrückt, sodass der Stromkreis geschlossen und der Messwert erzeugt wird. Insbesondere kann die Brücke je nach Partikelgröße durch genau einen ferromagnetischen Partikel oder durch eine Agglomeration mehrerer der ferromagnetischen Partikel in dem Messbereich bzw. innerhalb des Spaltes gebildet werden. Die elektrische Kenngröße kann insbesondere eine Impedanz sein. Insbesondere erfolgt bei einer Anordnung des mindestens einen ferromagnetischen Partikel zwischen den elektrischen Kontakten eine Änderung der Impedanz, wobei basierend auf der Impedanzänderung auf eine Menge und/oder Größe der ferromagnetischen Partikel rückgeschlossen werden kann. Vorzugsweise wird der Realteil und/oder der Imaginärteil der Impedanz ausgewertet. Es sei darauf hingewiesen, dass die elektrische Kenngröße der Kapazität, Frequenz, Konduktivität, oder dergleichen direkt oder indirekt detektiert werden kann.

Gemäss der Erfindung kann der Messwert anhand eines Auswertekriterium ausgewertet werden, um das Maß für die Kritikalität zu ermitteln. Beispielsweise kann das Auswertekriterium durch einen hinterlegten Sollwert oder Wertebereich definiert sein, mit welchem der erfasste Messwert verglichen wird. Beispielsweise kann im Rahmen der Auswertung der Messwert in einen vergleichbaren Wert umgewandelt werden. Alternativ kann jedoch auch der Messwert direkt ausgewertet werden. Insbesondere kann basierend auf dem Maß für die Kritikalität entschieden werden, ob eine Beschädigung des Getriebes und damit eine Gefährdung des Flugbetriebes vorliegt.

Der Vorteil der Erfindung liegt insbesondere darin, dass aufgrund des Maß für die Kritikalität, dem Piloten die Möglichkeit gegeben wird, selbständig während einer Mission die Entscheidung über einen Missionsabbruch treffen zu können. Zudem kann basierend auf dem Maß für die Kritikalität eine qualitative Aussage zum Zustand des Getriebes getroffen werden. Durch das Verfahren kann zudem in einfacher und kostengünstiger Weise eine Detektion von ferromagnetischen Partikeln in dem Getriebe realisiert werden, wobei zugleich durch eine Auswertung der elektrischen Kenngröße auf die Anzahl und Größe der detektierten Partikel rückgeschlossen werden kann.

Gemäss der Erfindung ist vorgesehen, dass basierend auf dem Maß für die Kritikalität ein Warnsignal erzeugt wird, wobei basierend auf dem Warnsignal ein Maß für die Kritikalität des Flugbetriebes angezeigt wird. Bevorzugt wird das Warnsignal in Echtzeit ausgegeben, wenn ein aktueller Messwert das Auswertekriterium erfüllt. Das Warnsignal kann beispielsweise als ein elektrisches Signal ausgegeben werden, welches zur weiteren Verarbeitung einer Rechen- und/oder Anzeigeeinrichtung bereitgestellt wird. Insbesondere kann das Maß für die Kritikalität optisch und/oder akustisch und/oder haptisch angezeigt werden. Es kann somit in einfacher Weise dem Piloten mitgeteilt werden, dass eine Gefährdung des Flugbetriebes vorliegt, wobei der Pilot auf Basis der Anzeige eine Entscheidung bezüglich der Mission treffen kann.

Gemäss der Erfindung ist vorgesehenen, dass das Maß für die Kritikalität in mindestens oder genau zwei Kritikalitätsstufen eingeteilt wird, wobei für jede der Kritikalitätsstufen ein entsprechendes Warnsignal generiert wird. Vorzugsweise kann über das Warnsignal eine Information bezüglich der Kritikalität übertragen werden. Insbesondere kann für jede Kritikalitätsstufe mindestens ein zugehöriges Auswertekriterium festgelegt sein, welches erfüllt sein muss, damit eine entsprechende Anzeige der Kritikalität erfolgt. Vorzugsweise werden die Kritikalitätsstufen auf Basis der Partikelgröße und/oder Partikelanzahl eingeteilt, welche sich basierend auf dem elektrischen Kennwert bzw. einer Änderung des elektrischen Kennwerts ermitteln lassen. Durch die stufenweise Anzeige der Kritikalität kann dem Piloten in einfacher Weise angezeigt werden, ob die Mission abgebrochen werden muss oder die Mission, ggf. eingeschränkt, beendet werden kann.

Gemäss der Erfindung ist vorgesehen, dass in einer ersten Kritikalitätsstufe keine Kritikalität, in einer zweiten Kritikalitätsstufe eine geringe Kritikalität und in einer dritten Kritikalitätsstufe eine hohe Kritikalität angezeigt wird. Dabei wird die erste Stufe angezeigt, wenn keine Partikel detektiert werden. Es wird die zweite Stufe angezeigt, wenn ein Partikel mit einer Partikelgröße kleiner oder gleich einer vorgegebenen maximal zulässigen Partikelgröße detektiert wird, z.B. größer als 10 Mikrometer und/oder kleiner als 50 Mikrometer. Weiter wird die dritte Stufe angezeigt, wenn mindestens ein Partikel mit einer großen Partikelgröße, z.B. größer als 50 Mikrometer, und/oder eine hohe Anzahl von Partikeln detektiert werden. Durch die unterschiedlichen Kritikalitätsstufen, kann eine Beschädigung des Getriebes zuverlässig eingestuft werden, sodass der Pilot ein Risiko des Flugbetriebes deutlich besser einschätzen kann.

In einer weiteren Umsetzung ist vorgesehen, dass das Maß für die Kritikalität des Flugbetriebes basierend auf mindestens einem weiteren Messwert bewertet wird, welcher unabhängig von dem Messwert erfasst wird. Insbesondere wird der mindestens ein weiterer Messwert zeitgleich oder simultan zu dem Messwert erfasst und mit dem Messwert korreliert, wobei eine Bewertung der Kritikalität in Abhängigkeit des korrelierten Messergebnisses erfolgt. Vorzugsweise erfolgt die Bewertung der Messwert anhand mindestens eines Bewertungskriteriums. Durch die Berücksichtigung mindestens eines weiteren Messwertes kann die Messsicherheit erhöht und zugleich eine Fehlinterpretation eines einzelnen Messwertes reduziert werden.

In einer Konkretisierung ist vorgesehen, dass eine geringe Kritikalität angezeigt wird, wenn der Messwert und/oder der weitere Messwert in die zweite Kritikalitätsstufe eingeteilt werden. Insbesondere ist für jeden Messwert ein separates Auswertekriterium hinterlegt, anhand dessen der Messwert in die jeweilige Kritikalitätsstufe eingeteilt wird. Vorzugsweise werden die Messwerte basierend auf einer Entscheidungslogik ausgewertet und/oder bewertet. Optional kann vorgesehen sein, dass die Messwerte unterschiedlich gewichtet werden. Beispielsweise kann die Gewichtung in Abhängigkeit eines aktuellen Betriebszustands und/oder der Messstelle und/oder der erkannten Partikel gewählt werden. So kann beispielsweise bei einer Detektion von Partikeln an typisch neuralgischen Punkten, eine hohe Kritikalität angezeigt, auch wenn der weitere Messwert das zugehörige Auswertekriterium nicht erfüllt bzw. in die erste oder zweite Kritikalitätsstufe eingeteilt wird. Des Weiteren wird eine hohe Kritikalität angezeigt, wenn der Messwert und/oder der weitere Messwert in die dritte Kritikalitätsstufe eingeteilt werden. Insbesondere wird eine hohe Kritikalität angezeigt, wenn mindestens zwei oder alle Messwerte in die dritte Kritikalitätsstufe eingeteilt werden. Optional können ebenfalls unterschiedliche Gewichtungen berücksichtigt werden. Es wird somit ein Verfahren vorgeschlagen, welches unterschiedliche Messwerte miteinander vergleicht und bewertet, sodass die Messsicherheit deutlich erhöht wird.

In einer weiteren Konkretisierung ist vorgesehen, dass der weitere Messwert bei einer Erkennung mindestens eines weiteren ferromagnetischen Partikels in einem weiteren Messbereich in dem Getriebe ausgegeben wird. Insbesondere kann der weitere Messbereich in dem Getriebeabschnitt oder einem weiteren Getriebeabschnitt angeordnet sein. Bevorzugt erfolgt in einem weiteren Messbereich eine Detektion von ferromagnetischen Partikeln, wie dies bereits zuvor beschrieben wurde. Insbesondere kann eine Detektion von ferromagnetischen Partikeln an mindestens oder genau zwei, vorzugsweise mehr als drei, im Speziellen mehr als fünf unterschiedlichen Positionen bzw. Messbereichen in dem Getriebe stattfinden. Alternativ oder optional ergänzend wird der weitere Messwert bei einer Änderung eines Getriebeparameters ausgegeben. Beispielsweise können hierzu ein oder mehrere bekannte Getriebeparameter wie Vibrationen, Geräusche, Drehzahl, Drehmoment, Getriebetemperatur oder dergleichen ausgewertet werden. Es wird somit ein Verfahren vorgeschlagen, welches sich durch eine hohe Messgenauigkeit auszeichnet, wobei unter Berücksichtigung mehrerer Messwerte ein Ausfall des Getriebes frühzeitig und zuverlässig erkannt werden kann.

Es ist bevorzugt vorgesehen, dass die elektrische Kenngröße ein ohmscher Widerstand ist. Insbesondere weisen Partikel mit einem kleinen Querschnitt einen höheren Widerstand als Partikel mit einem größeren Querschnitt auf. Beispielsweise kann basierend auf dem ohmschen Gesetz der ohmsche Widerstand ermittelt und als Messwert bereitgestellt werden. Somit kann auf Basis des ohmschen Widerstands die Partikelgröße ermittelt werden. Alternativ oder optional ergänzend ist die elektrische Kenngröße eine elektrische Spannung. Insbesondere fällt eine Spannung an der elektrisch leitfähigen Brücke ab, sodass die Spannungsdifferenz zur Bildung des Messwertes herangezogen werden kann. Beispielsweise kann die elektrische Spannung basierend auf dem ohmschen Gesetz ermittelt werden. Somit kann auf Basis der Spannung bzw. einer Änderung der Spannung die Partikelgröße ermittelt werden.

Ein weiterer Gegenstand der Erfindung bildet ein Überwachungssystem, welches zur Überwachung eines Getriebes in einem Luftfahrzeug ausgebildet und/oder geeignet ist. Dabei ist das Überwachungssystem zur Durchführung des Verfahrens, wie dies bereits zuvor beschrieben wurde, ausgebildet und/oder geeignet.

Das Überwachungssystem weist mindestens oder genau einem Partikelerkennungssensor auf, welcher zur Erkennung von ferromagnetischen Partikeln in einem Messbereich des Getriebes ausgebildet ist. Insbesondere ist der Partikelerkennungssensor in dem Getriebeabschnitt des Getriebes angeordnet, wobei der Partikelerkennungssensor den Messbereich definiert. Der Messbereich ist dabei als ein Bereich um den Sensor zu verstehen, innerhalb dessen die ferromagnetischen Partikeln erkannt werden. Der Partikelerkennungssensor weist hierzu zwei voneinander beanstandete elektrische Kontakte auf, welche innerhalb des Messbereichs angeordnet und an einen elektrischen Stromkreis angeschlossen sind. Bevorzugt ist der Partikelerkennungssensor als ein Spanerkennungssensor ausgebildet, welcher zur Detektion von ferromagnetischer und insbesondere metallischer Späne ausgebildet und/oder geeignet ist. Derartige Sensoren sind unteranderem auch als magnetische Abriebdetektoren (Magnetic Chip Detectors) bekannt.

Der Partikelerkennungssensor weist einen Magneten auf, welcher zur Erzeugung eines Magnetfelds ausgebildet und/oder geeignet ist. Der Magnet kann dabei als ein Permanentmagnet oder als ein Elektromagnet ausgebildet sein. Der Magnet hat die Funktion, ein oder mehrere in dem Getrieberaum angeordnete ferromagnetische Partikel in den Messbereich des Partikelerkennungssensors zu bewegen, sodass sich eine elektrische leitfähige Brücke aus ein oder mehreren der ferromagnetischen Partikeln zwischen den beiden elektrischen Kontakten bildet. Der Magnet ist vorzugsweise derart angeordnet, sodass ein Wirkbereich des Magneten, in dem die ferromagnetischen Partikel magnetisch angezogen werden, zwischen den beiden Kontakten und/oder in dem Messbereich angeordnet ist. Beispielsweise kann der Magnet hierzu zwischen den beiden Kontakten in dem Spalt angeordnet sein. Bei Vorhandensein von ein oder mehreren der ferromagnetischen Partikel zwischen den elektrischen Kontakten, sind auf Basis der elektrischen Kenngröße zwischen den beiden Kontakten detektierbar. Der Partikelerkennungssensor ist dabei ausgebildet, auf Basis einer gemessenen elektrischen Kenngröße, einen Messwert auszugeben. Insbesondere ist der Messwert als ein elektrisches Messsignal ausgebbar.

Das Überwachungssystem weist zudem eine Auswerteeinrichtung auf, welche zur Auswertung des Messwertes ausgebildet und/oder geeignet ist, um ein Maß für die Kritikalität des Flugbetriebs durch eine Auswertung des Messwertes zu ermitteln. Vorzugsweise sind der Partikelerkennungssensor und die Auswerteeinrichtung signaltechnisch miteinander verbunden. Insbesondere ist in der Auswerteeinrichtung mindestens ein Auswertekriterium hinterlegt, anhand dessen der Messwert in das Maß für die Kritikalität abgebildet wird. Beispielsweise kann die Auswerteeinrichtung als ein Hardware- oder Softwaremodul in einem Flugrechner ausgebildet sein. Die Auswerteeinrichtung ist ausgebildet.

In einer weiteren Ausgestaltung ist vorgesehen, dass das Überwachungssystem mindestens oder genau einen weiteren Partikelerkennungssensor aufweist, welcher zum Erkennen von ferromagnetischen Partikeln in einem weiteren Messereich des Getriebes ausgebildet ist. Insbesondere sind der Messbereich und der weitere Messbereich an unterschiedlichen Positionen des Getriebes, insbesondere in unterschiedlichen und/oder voneinander getrennten Getriebeabschnitten angeordnet. Alternativ können die beiden Messbereiche jedoch auch benachbart zueinander angeordnet, insbesondere in einem gemeinsamen Getriebeabschnitt, angeordnet sein. Hierzu ist der weitere Partikelerkennungssensor wahlweise in dem Getriebeabschnitt oder dem weiteren Getriebeabschnitt angeordnet. Insbesondere sind der Partikelerkennungssensor und der weitere Partikelerkennungssensor baugleich ausgebildet. Beispielsweise kann das Überwachungssystem mehr als zwei, insbesondere mehr als vier, im Speziellen mehr als sechs der Partikelerkennungssensoren aufweisen. Der weitere Partikelerkennungssensor ist ausgebildet, bei Erkennen mindestens eines weiteren ferromagnetischen Partikels einen weiteren Messwert auszugeben. Bevorzugt sind alle Partikelerkennungssensoren signaltechnisch mit der Auswerteeinrichtung verbunden. Die Auswerteeinrichtung ist ausgebildet, ein Maß für die Kritikalität des Flugbetriebs basierend auf dem Messwert und dem weiteren Messwert zu bewerten. Insbesondere ist die Auswerteeinrichtung ausgebildet, die Kritikalität anhand einer Entscheidungslogik zu bewerten.

In einer alternativen oder optional ergänzenden Ausführung ist vorgesehen, dass das Überwachungssystem mindestens oder genau einen Getriebeüberwachungssensor aufweist, welcher zur Erfassung eines Getriebeparameters ausgebildet und/oder geeignet ist. Prinzipiell kann der Getriebeüberwachungssensor als ein Temperatur-, Drehzahl- oder Drehmomentsensor ausgebildet sein. Bevorzugt jedoch ist der Getriebeüberwachungssensor als ein Beschleunigungssensor ausgebildet, welcher zur Erfassung von Vibrationen in dem Getriebe geeignet ist. Der Getriebeüberwachungssensor ist ausgebildet, den Getriebeparameter als einen weiteren Messwert auszugeben und an die Auswerteeinheit zu übermitteln. Hierzu sind der Getriebeüberwachungssensor und die Auswerteeinrichtung signaltechnisch miteinander verbunden. Die Auswerteeinrichtung ist ausgebildet, ein Maß für die Kritikalität des Flugbetriebs durch eine Auswertung des elektrischen Messwertes und des Getriebeparameters zu bewerten. Im Speziellen können die Sensordaten mehrerer Getriebeüberwachungssensoren genutzt und mit den Messwerten des mindestens einen Partikelerkennungssensors korreliert werden, um die Kritikalität zu bewerten.

In einer weiteren Ausführung ist vorgesehen, dass das Überwachungssystem eine Anzeigeeinrichtung aufweist, welche ausgebildet ist, ein Maß für die Kritikalität des Flugbetriebs anzuzeigen. Insbesondere ist die Anzeigeeinrichtung in einem Sichtbereich des Piloten, vorzugsweise innerhalb des Cockpits, angeordnet und/oder anordbar. Die Anzeigeeinrichtung ist vorzugsweise als ein Leuchtmittel oder ein Display ausgebildet, welches zur Anzeige der Kritikalität mindestens zwei unterschiedliche Anzeigezustände einnehmen kann. Beispielsweise können die Anzeigezustände durch das Anzeigen unterschiedlicher Lichtsignale und/oder Meldungen voneinander unterschieden werden.

In einer konkreten Ausgestaltung ist vorgesehen, dass zwischen den beiden Kontakten ein v-förmiger Spalt gebildet ist, welcher dazu eingerichtet ist, mehrere der ferromagnetischen Partikel anzuhäufen. Insbesondere sind die beiden Kontakte mit einem Winkel von mehr als 20 Grad, vorzugsweise mehr als 50 Grad, im Speziellen mehr als 80 Grad zueinander angeordnet. Durch den v-förmigen Spalt können ferromagnetische Partikel unterschiedlicher Größe angehäuft werden. Dabei kann bei einer Messung über die Zeit auf einen normalen Abrieb oder einen kritischen Verschleiß rückgeschlossen werden.

Ein weiterer Gegenstand der Erfindung betrifft ein Luftfahrzeug mit dem Überwachungssystem, wie dies bereits zuvor beschrieben wurde. Das Luftfahrzeug kann prinzipiell als ein Flugzeug, Lufttaxi oder dergleichen ausgebildet ein. Bevorzugt jedoch ist das Luftfahrzeug als ein Helikopter ausgebildet.

Weitere Merkmale, Wirkungen und Vorteile der Erfindung ergeben sich aus der nachfolgenden Beschreibung eines bevorzugten Ausführungsbeispiels der Erfindung sowie der beigefügten Figuren. Dabei zeigen:
- Figur 1: eine stark schematische Darstellung eines Luftfahrzeugs mit einem Überwachungssystem als ein Ausführungsbeispiel der Erfindung;
- Figur 2: eine schematische Darstellung eines Partikelerkennungssensors des Überwachungssystems gemäß Figur 1 ;
- Figur 3: den Partikelerkennungssensor in gleicher Darstellung wie in Figur 2 in einem ersten Detektionszustand;
- Figur 4: den Partikelerkennungssensor in gleicher Darstellung wie in Figur 2 in einem zweiten Detektionszustand;
- Figur 5: den Partikelerkennungssensor in gleicher Darstellung wie in Figur 2 in einem dritten Detektionszustand;
- Figur 6: eine Entscheidungslogik zur Bewertung einer Kritikalität des Flugbetriebes anhand eines schematischen Blockdiagramms.

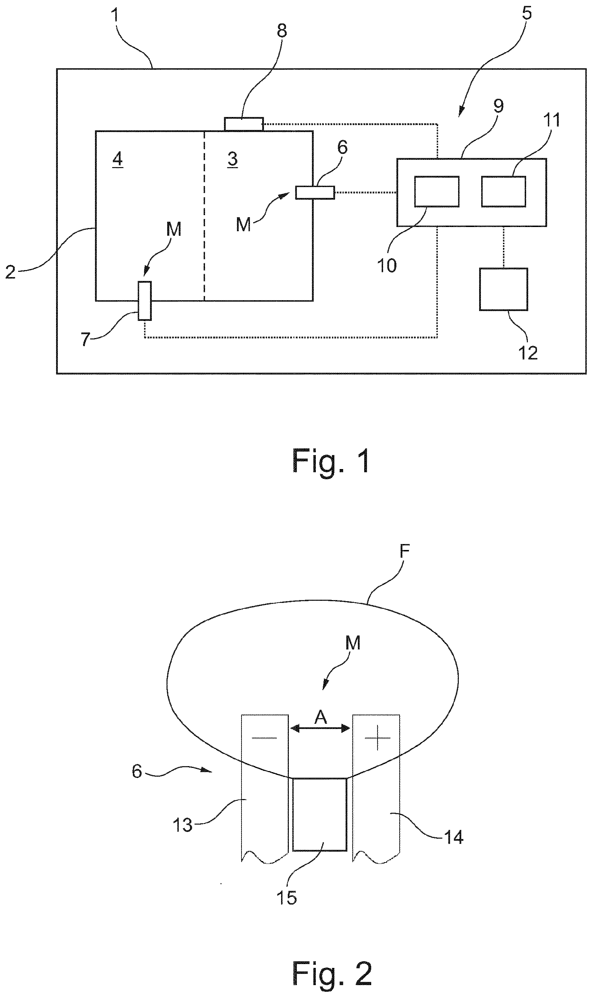

Figur 1 zeigt in einer stark schematisierten Blockdarstellung ein Luftfahrzeug 1, hier nur als Funktionsblock dargestellt, als ein Ausführungsbeispiel der Erfindung. Beispielsweise ist das Luftfahrzeug 1 als ein Helikopter ausgebildet.

Das Luftfahrzeug 1 weist ein Getriebe 2 auf, welches zur Übersetzung eines Antriebsmoments dient. Beispielsweise kann das Getriebe 2 das Antriebsmoment von einem Motor auf ein Triebwerk und/oder auf ein oder mehrere Rotoren übersetzen.

Das Getriebe 2 ist bevorzugt als ein mechanisches Getriebe, z.B. ein Zahnradgetriebe, ausgebildet, welches während eines Flugbetriebes hohen Momenten und Drehzahlen ausgesetzt ist.

Das Getriebe 2 kann in mehrere Getriebeabschnitte 3, 4 unterteilt sein, welche beispielsweise als Teilabschnitte eines Getriebeinnenraums des Getriebes 2 ausgebildet sind. Beispielsweise können die Getriebeabschnitte 3, 4 räumlich voneinander getrennt sein, z.B. Nass- und Trockenraum. Alternativ können die Getriebeabschnitte 3, 4 jedoch auch einen gemeinsamen Innenraum des Getriebes 2 definieren.

Das Luftfahrzeug 1 weist ein Überwachungssystem 5 auf, welches zur Überwachung des Getriebes 2 während eines Flugbetriebes dient. Das Überwachungssystem 5 weist hierzu mindestens einen Partikelerkennungssensor 6 auf, welcher zur Erkennung von ferromagnetischen Partikeln dient. Diese ferromagnetischen Partikel können sich beispielsweise infolge eines Abriebs oder auch durch Schädigungen der Getriebekomponenten in Folge der hohen Belastungen bilden. Der Partikelerkennungssensor 6 ist dabei in dem ersten Getriebeabschnitt 3 angeordnet, um eventuell darin angeordnete ferromagnetische Partikel zu detektieren. Dabei gibt der Partikelerkennungssensor 6 bei einer Detektion eines oder mehrerer ferromagnetischen Partikel einen Messwert aus.

Optional kann das Überwachungssystem 5 einen weiteren Partikelerkennungssensor 7 aufweisen, welcher zur Erkennung von ferromagnetischen Partikeln in dem zweiten Getriebeabschnitt 4 angeordnet ist. Durch den weiteren Partikelerkennungssensor 7 können somit ferromagnetische Partikel an unterschiedlichen Positionen in dem Getriebe 2 erkannt werden. Dabei gibt der weitere Partikelerkennungssensor 7 bei einer Detektion eines oder mehrerer ferromagnetischen Partikel einen weiteren Messwert aus.

Alternativ oder optional ergänzend kann das Überwachungssystem 5 einen Getriebeüberwachungssensor 8 aufweisen, welcher zur Erfassung eines Getriebeparameters dient. Beispielsweise ist der Getriebeüberwachungssensor 8 als ein Beschleunigungssensor ausgebildet, welcher zur Erfassung von Vibrationen in oder an dem Getriebe 2 angeordnet ist. Dabei gibt der Getriebeüberwachungssensor 8 den Getriebeparameter als einen weiteren Messwert aus.

Das Überwachungssystem 5 weist eine Auswerteeinrichtung 9 auf, welche zur Auswertung der Messwerte eingerichtet ist. Die Sensoren 6, 7, 8 sind hierzu signaltechnisch mit der Auswerteinrichtung 9 verbunden. Beispielsweise bildet die Auswerteeinrichtung 9 einen integralen Bestandteil eines Flugrechners.

Die Auswerteeinrichtung 9 weist ein Auswertemodul 10 auf, wobei das Auswertemodul 10 ausgebildet ist, die Messwerte anhand mindestens eines Auswertekriteriums auszuwerten, um ein Maß für die Kritikalität des Flugbetriebes zu ermitteln. Des Weiteren kann die Auswerteeinrichtung 9 ein Bewertungsmodul 11 aufweisen, welches ausgebildet ist, die Messwerte der Sensoren 6, 7, 8 miteinander zu korrelieren und die Kritikalität auf Basis eines Bewertungskriteriums zu bewerten.

Die Auswerteeinrichtung 10 ist ausgebildet, ein Warnsignal auszugeben, wenn mindestens ein Auswertekriterium und/oder Bewertungskriterium erfüllt ist, wobei das Warnsignal einer Anzeigeeinrichtung 12 bereitgestellt wird. Die Anzeigeeinrichtung 12 kann beispielsweise als ein Leuchtmittel ausgebildet sein, wobei die Anzeigeeinrichtung 13 das Warnsignal in ein Lichtsignal umwandelt, um das Maß für die Kritikalität des Flugbetriebes anzuzeigen. Die Anzeigeeinrichtung 10 ist hierzu vorzugsweise in einem Sichtbereich des Piloten angeordnet, wobei der Pilot auf Basis des Lichtsignals entscheidet, ob die Mission abgebrochen werden muss oder ob die Mission beendet werden kann.

Die Figur 2 zeigt in einer schematischen Darstellung den Partikelerkennungssensor 6 als ein weiteres Ausführungsbeispiel der Erfindung. Es sei darauf hingewiesen, dass die nachfolgende Beschreibung der Funktion analog auf den weiteren Partikelerkennungssensor 7 anwendbar ist.

Der Partikelerkennungssensor 6 weist einen ersten und einen zweiten elektrischen Kontakt 13, 14 auf, welche über einen Abstand A elektrisch voneinander getrennt sind und innerhalb des jeweiligen Getriebeabschnitts 3, 4 angeordnet sind. Die beiden Kontakte 13, 14 sind an einen elektrischen Stromkreis angeschlossen, welcher im Bereich der Kontakte 13, 14 durch den gebildeten Spalt zwischen Kontakten 13, 14 unterbrochen ist.

Der Partikelerkennungssensor 6 weist einen Magneten 15 auf, welcher ein Magnetfeld F innerhalb des Getriebeabschnitts 3 erzeugt. Beispielsweise kann der Magnet 15 als ein Permanentmagnet oder als ein Elektromagnet ausgebildet sein. Der Magnet 15 ist bevorzugt zwischen den beiden Kontakten 13, 14 angeordnet, sodass ein ein in dem Getriebeabschnitt 3 angeordneter ferromagnetischer Partikel aufgrund der magnetischen Wechselwirkung in einen Messbereich M des Partikelerkennungssensors 6 bewegt wird. Der Messbereich 5 ist dabei als der Bereich des Partikelerkennungssensors 6 zu verstehen, an welchem die ferromagnetischen Partikel mit den Kontakten 13, 14 kontaktieren oder sich an diesen anlagern können.

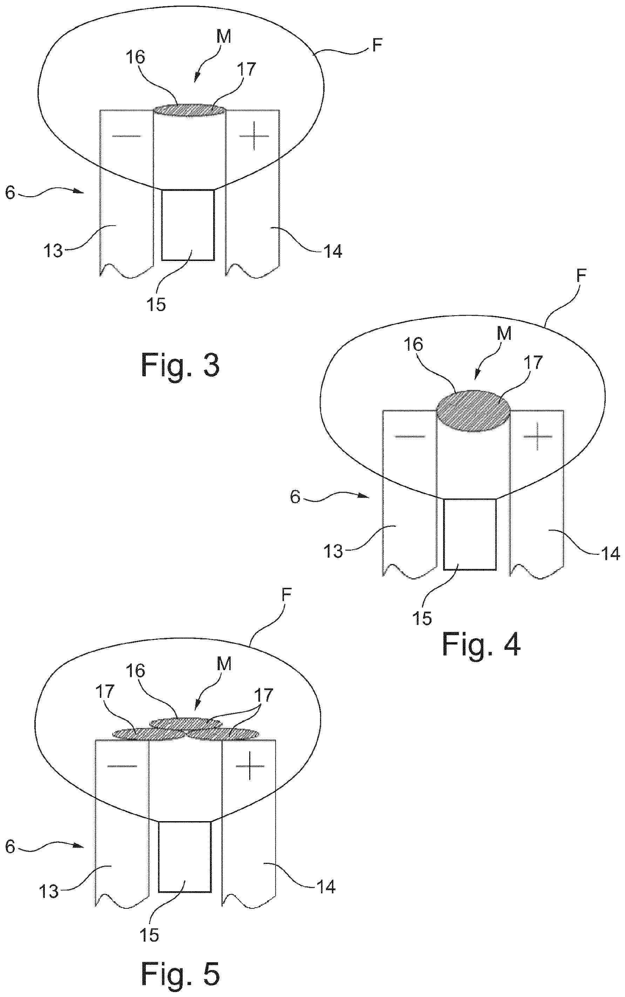

Die Figuren 3 bis 5 zeigen jeweils die Erkennung unterschiedlicher Partikelgrößen bzw. -anzahl durch den Partikelerkennungssensor 6, wie dieser bereits in Figur 2 beschrieben wurde. In einem Detektionszustand ist mindestens ein Partikel 17 zwischen den beiden Kontakten 13, 14 angeordnet, wobei das Vorhandensein eines oder mehrerer Partikel 17 basierend auf einer elektrischen Kenngröße, insbesondere eine Impedanzänderung, zwischen den beiden Kontakten 13, 14 detektierbar ist. Bevorzugt wird in Abhängigkeit der Partikelgröße und/oder Anzahl eine elektrische leitfähige Brücke 16 aus ein oder mehreren Partikeln 17 gebildet, welche die beiden Kontakte 13, 14 überbrückt, sodass der Stromkreis geschlossen wird.

Der von dem Partikelerkennungssensor 6 ausgegebene Messwert wird anhand eines in dem Auswertemodul 10 hinterlegten Auswertekriteriums ausgewertet, um ein Maß für die Kritikalität des Flugbetriebes zu bestimmen. Dabei kann die elektrische Kenngröße beispielsweise ein ohmscher Widerstand oder eine Spannung sein, welche nach dem ohmschen Gesetz ausgewertet werden. Das Maß für die Kritikalität wird dabei auf Basis der detektierten Partikelgröße und/oder -anzahl bestimmt. Beispielsweise weist ein Partikel 17 mit einem kleinen Querschnitt einen höheren elektrischen Widerstand auf, als ein Partikel 17 mit einem größeren Querschnitt, sodass basierend auf den elektrischen Widerstand auf die Partikelgröße zurückgeschlossen werden kann. Zusätzlich kann in Abhängigkeit der Zeit darauf zurückgeschlossen werden, wie viele Partikel 17 innerhalb eines festgelegten Zeitraums sich an dem Partikelerkennungssensor 6 angelagert haben.

Beispielsweise ist die Auswerteeinrichtung 9 ausgebildet, das Maß für die Kritikalität in drei Stufen zu unterteilen, wobei in einer ersten Stufe keine Kritikalität, in einer zweiten Stufe eine geringe Kritikalität und in einer dritten Stufe eine hohe Kritikalität durch die Anzeigeeinrichtung 12 angezeigt wird. Die Einteilung der Stufen erfolgt dabei in Abhängigkeit der ermittelten Partikelgröße bzw. Anzahl. Beispielsweise wird die erste Stufe angezeigt, wenn keine Partikel 17 durch den Partikelerkennungssensor 6 detektiert werden. Beispielsweise wird die zweite Stufe angezeigt, wenn ein einzelner Partikel 17 mit einer maximal zulässigen Partikelgröße, z.B. kleiner oder gleich als 50 Mikrometer, detektiert wird, wie dies in Figur 3 gezeigt ist. Beispielsweise wird die dritte Stufe angezeigt, wenn ein Partikel 17 detektiert wird, der größer als die maximal zulässige Partikelgröße ist, wie in Figur 4 gezeigt, und/oder wenn mehrere Partikel 17 innerhalb des festgelegten Zeitraums an dem Partikelerkennungssensor 6 detektiert werden, wie in Figur 5 gezeigt. Beispielsweise kann die Anzeigeeinrichtung 12 ausgebildet sein, die einzelnen Stufen als unterschiedliche Farben, z.B. Ampelfarben, anzuzeigen. Beispielsweise kann die erste Stufe als grünes Leuchtsignal, die zweite Stufe als gelbes Leuchtsignal und die dritte Stufe als rotes Leuchtsignal angezeigt werden. Dadurch wird dem dem Piloten die Möglichkeit geben zu entscheiden, ob ein Abbruch des Flugbetriebes erforderlich ist.

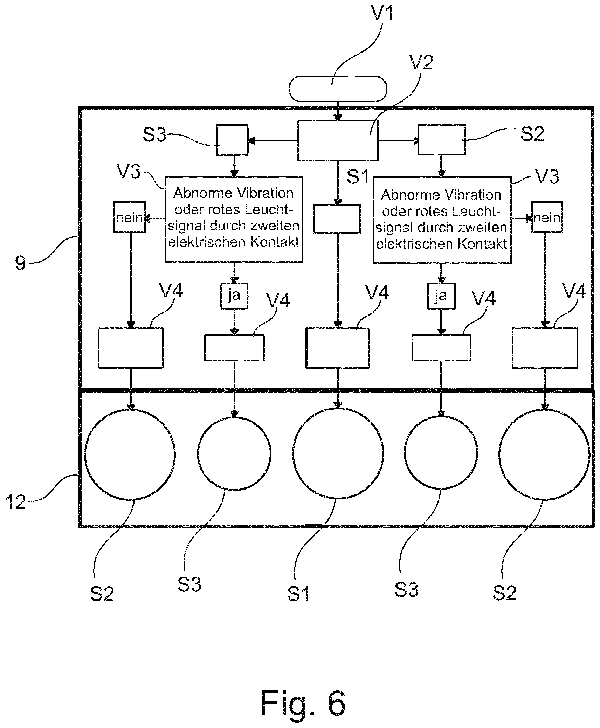

Die Figur 6 zeigt anhand eines Ablaufdiagram eine Entscheidungslogik zur Bewertung der Kritikalität während eines Flugbetriebes.

In einem ersten Verfahrensschritt V1 wird der Messwert des Partikelerkennungssensors 6 der Auswerteeinrichtung 9 bereitgestellt und ggf. in ein auswertbaren Wert konvertiert.

In einem zweiten Verfahrensschritt V2 wird der Messwert ausgewertet und in eine der drei Kritikalitätsstufen S1, S2, S3 eingeteilt.

Um eine Fehlinterpretation des Messwertes zu vermeiden, wird der Messwert des weiteren Partikelerkennungssensors 7 und/oder des Getriebeüberwachungssensors 8 in einem dritten Verfahrensschritt V3 hinzugefügt und in eine der drei Kritikalitätsstufen S1, S2, S3 eingeteilt, wenn der Messwert des Partikelerkennungssensors 6 in dem zweiten Verfahrensschritt V2 in die zweite oder dritte Kritikalitätsstufe S2, S3 eingeteilt wird.

In einem vierten Verfahrensschritt V4 wird auf Basis der jeweiligen Kritikalitätsstufe S1, S2, S3 ein Warnsignal generiert und der Anzeigeeinrichtung 12 bereitgestellt, wobei die Anzeigeeinrichtung 12 ausgebildet ist, die jeweilige Kritikalitätsstufe S1, S2, S3 auf Basis des Warnsignals anzuzeigen. Dabei zeigt die Anzeigeeinrichtung 12 solange die erste Stufe S1 an, bis ein Partikel 17 durch den Partikelerkennungssensor 6 detektiert wird.

Wird der Messwert des Partikelerkennungssensors 6 im Rahmen des zweiten Verfahrensschritts V2 in die zweite oder dritte Kritikalitätsstufe S2, S3 und der Messwert des weiteren Partikelerkennungssensors 7 und/oder des Getriebeüberwachungssensors 8 im Rahmen des dritten Verfahrensschritts V3 in die dritte Kritikalitätsstufe S3 eingeteilt, wird im Rahmen des vierten Verfahrensschritt V4 ein Warnsignal zur Anzeige der dritten Kritikalitätsstufe S3 erzeugt.

Wird hingegen der Messwert des Partikelerkennungssensors 6 im Rahmen des zweiten Verfahrensschritts V2 in die zweite oder dritte Stufe S2, S3 und der Messwert des weiteren Partikelerkennungssensors 7 und/oder des Getriebeüberwachungssensors 8 im Rahmen des dritten Verfahrensschritts V3 in die zweite Kritikalitätsstufe S3 eingeteilt, wird im Rahmen des vierten Verfahrensschritt V4 ein Warnsignal zur Anzeige der zweiten Kritikalitätsstufe S2 erzeugt. Somit erhält der Pilot eine qualitative Aussage zum Zustand des Getriebes 2, wobei der Pilot auf Basis der Anzeige entscheiden kann ob er den Flug beenden kann oder unterbrechen muss.

### Bezugszeichen

- 1: Luftfahrzeug
- 2: Getriebe
- 3: erster Getriebeabschnitt
- 4: zweiter Getriebeabschnitt
- 5: Überwachungssystem
- 6: Partikelerkennungssensor
- 7: weiterer Partikelerkennungssensor
- 8: Getriebeüberwachungssensor
- 9: Auswerteeinrichtung
- 10: Auswertemodul
- 11: Bewertungsmodul
- 12: Anzeigeeinrichtung
- 13: erster Kontakt
- 14: zweiter Kontakt
- 15: Magnet
- 16: leitfähige Brücke
- 17: ferromagnetischer Partikel

- A: Abstand
- F: Magnetfeld
- M: Messbereich
- V1-V4: Verfahrensschritte
- S1-S3: Kritikalitätsstufen

## Patentansprüche

1. Verfahren zur Überwachung eines Getriebes (2) in einem Luftfahrzeug (1), wobei das Getriebe (2) mindestens einen Getriebeabschnitt (3) mit mindestens einem Messbereich (M) aufweist, wobei in dem Messbereich (M) ein erster und ein zweiter elektrischer Kontakt (13, 14) mit einem Abstand (A) zueinander angeordnet sind, bei dem:
- ein Magnetfeld (F) in dem Getriebeabschnitt (3) erzeugt wird, wobei das Magnetfeld (F) dazu eingerichtet ist, mindestens einen in dem Getriebeabschnitt (3) angeordneten ferromagnetischen Partikel (17) zum Messbereich (M) hin zu bewegen, sodass sich eine Brücke (16) aus ein oder mehreren der ferromagnetischen Partikeln (17) zwischen den beiden elektrischen Kontakten (13, 14) bildet;
- basierend auf einer elektrischen Kenngröße zwischen den beiden elektrischen Kontakten (13, 14) ein Messwert erzeugt wird;
- basierend auf dem erzeugten Messwert von einem Auswertemodul (10) ein Maß für die Kritikalität eines Flugbetriebs ermittelt wird, wobei basierend auf dem Maß für die Kritikalität ein Warnsignal erzeugt wird und basierend auf dem Warnsignal das Maß für die Kritikalität des Flugbetriebes angezeigt wird, wobei das Maß für die Kritikalität in mindestens zwei Kritikalitätsstufen (S1, S2, S3) eingeteilt wird und für jede der Kritikalitätsstufen (S1, S2, S3) ein entsprechendes Warnsignal generiert wird, und wobei in einer ersten Kritikalitätsstufe (S1) keine Kritikalität angezeigt wird, in einer zweiten Kritikalitätsstufe (S2) eine geringe Kritikalität angezeigt wird und in einer dritten Kritikalitätsstufe (S3) eine hohe Kritikalität angezeigt wird,
**dadurch gekennzeichnet, dass** anhand eines in dem Auswertemodul (10) hinterlegten Auswertekriteriums eine detektierte Partikelgröße und/oder -anzahl für den erzeugten Messwert bestimmt wird, wobei das Maß für die Kritikalität in die erste Kritikalitätsstufe (S1) eingestuft wird, wenn die detektierte Partikelanzahl innerhalb eines festgelegten Zeitraums gleich null ist, wobei das Maß für die Kritikalität in die zweite Kritikalitätsstufe (S2) eingestuft wird, wenn die detektierte Partikelgröße kleiner oder gleich einer vorgegebenen maximal zulässigen Partikelgröße ist und die detektierte Partikelanzahl innerhalb des festgelegten Zeitraums gleich eins ist, und wobei das Maß für die Kritikalität in die dritte Kritikalitätsstufe (S3) eingestuft wird, wenn die detektierte Partikelgröße größer als die vorgegebene maximal zulässige Partikelgröße ist und/oder die detektierte Partikelanzahl innerhalb des festgelegten Zeitraums größer als eins ist.

2. Verfahren nach Anspruch 1, **dadurch gekennzeichnet, dass** das Maß für die Kritikalität basierend auf mindestens einem weiteren Messwert bewertet wird, welcher unabhängig von dem erzeugten Messwert erfasst wird.

3. Verfahren nach Anspruch 2, **dadurch gekennzeichnet, dass** eine geringe Kritikalität angezeigt wird, wenn der erzeugte Messwert und/oder der weitere Messwert in die zweite Kritikalitätsstufe (S2) eingeteilt werden, und dass eine hohe Kritikalität angezeigt wird, wenn sowohl der erzeugte Messwert und/oder der weitere Messwert in die dritte Kritikalitätsstufe (S3) eingeteilt werden.

4. Verfahren nach Anspruch 2 oder 3, **dadurch gekennzeichnet, dass** der weitere Messwert bei einer Erkennung mindestens eines weiteren ferromagnetischen Partikels (17) in einem weiteren Messbereich (M) des Getriebes (2) ausgegeben wird und/oder bei einer Änderung eines Getriebeparameters des Getriebes (2) erzeugt wird.

5. Verfahren nach einem der vorhergehenden Ansprüche, **dadurch gekennzeichnet, dass** die elektrische Kenngröße ein ohmscher Widerstand und/oder eine Spannung ist.

6. Überwachungssystem (5) für ein Getriebe (2) in einem Luftfahrzeug (1),
mit mindestens einem Partikelerkennungssensor (6) zur Erkennung von ferromagnetischen Partikeln (17) in einem Messbereich (M),
- wobei der Partikelerkennungssensor (6) in einem Getriebeabschnitt (3) des Getriebes (2) angeordnet ist,
- wobei der Partikelerkennungssensor (6) zwei voneinander beabstandete elektrische Kontakte (13, 14) aufweist,
- wobei der Partikelerkennungssensor (6) einen Magneten (15) zur Erzeugung eines Magnetfelds (F) aufweist, wobei der Magnet (15) ausgebildet ist, einen in dem Getriebeabschnitt (3) angeordneten ferromagnetischen Partikel in den Messbereich (M) des Partikelerkennungssensors (6) zu bewegen, sodass sich eine elektrische leitfähige Brücke (16) aus ein oder mehreren der ferromagnetischen Partikeln (17) zwischen den beiden elektrischen Kontakten (13, 14) bildet,
- wobei der Partikelerkennungssensor (6) ausgebildet ist, einen Messwert auf Basis einer elektrischen Kenngröße zwischen den beiden elektrischen Kontakten (13, 14) auszugeben,
mit einer Auswerteeinrichtung (9) zur Auswertung des Messwertes,
- wobei die Auswerteeinrichtung (9) ausgebildet ist, ein Maß für die Kritikalität des Flugbetriebs durch eine Auswertung des Messwertes zu ermitteln, wobei basierend auf dem Maß für die Kritikalität von der Auswerteeinrichtung (9) ein Warnsignal erzeugt wird und basierend auf dem Warnsignal das Maß für die Kritikalität des Flugbetriebes angezeigt wird, wobei das Maß für die Kritikalität in mindestens zwei Kritikalitätsstufen (S1, S2, S3) eingeteilt wird und für jede der Kritikalitätsstufen (S1, S2, S3) ein entsprechendes Warnsignal generiert wird, und wobei in einer ersten Kritikalitätsstufe (S1) keine Kritikalität angezeigt wird, in einer zweiten Kritikalitätsstufe (S2) eine geringe Kritikalität angezeigt wird und in einer dritten Kritikalitätsstufe (S3) eine hohe Kritikalität angezeigt wird,
**dadurch gekennzeichnet, dass** die Auswerteeinrichtung (9) ein Auswertemodul (10) aufweist, in dem ein Auswertekriterium hinterlegt ist, um anhand des Auswertekriteriums eine detektierte Partikelgröße und/oder -anzahl für den Messwert zu bestimmen, wobei das Maß für die Kritikalität in die erste Kritikalitätsstufe (S1) eingestuft wird, wenn die detektierte Partikelanzahl innerhalb eines festgelegten Zeitraums gleich null ist, wobei das Maß für die Kritikalität in die zweite Kritikalitätsstufe (S2) eingestuft wird, wenn die detektierte Partikelgröße kleiner oder gleich einer vorgegebenen maximal zulässigen Partikelgröße ist und
die detektierte Partikelanzahl innerhalb des festgelegten Zeitraums gleich eins ist, und wobei das Maß für die Kritikalität in die dritte Kritikalitätsstufe (S3) eingestuft wird, wenn die detektierte Partikelgröße größer als die vorgegebene maximal zulässige Partikelgröße ist und/oder die detektierte Partikelanzahl innerhalb des festgelegten Zeitraums größer als eins ist.

7. Überwachungssystem (5) nach Anspruch 6, **gekennzeichnet durch** mindestens einen weiteren Partikelerkennungssensor (7) zum Erkennen von ferromagnetischen Partikeln (17) in einem weiteren Messereich (M) des Getriebes (2), wobei der weitere Partikelerkennungssensor (7) in dem Getriebeabschnitt (3) oder einem weiteren Getriebeabschnitt (4) angeordnet ist, wobei der weitere Partikelerkennungssensor (7) ausgebildet ist, bei Erkennen mindestens eines weiteren ferromagnetischen Partikels (17) einen weiteren Messwert auszugeben, wobei die Auswerteeinrichtung (9) ausgebildet ist, ein Maß für die Kritikalität des Flugbetriebs basierend auf dem Messwert und dem weiteren Messwert zu bewerten.

8. Überwachungssystem (5) nach Anspruch 6 oder 7, **gekennzeichnet durch** mindestens einen Getriebeüberwachungssensor (8) zur Erfassung eines Getriebeparameters des Getriebes (2), wobei der Getriebeüberwachungssensor (8) ausgebildet ist, den Getriebeparameter als einen weiteren Messwert auszugeben, wobei die Auswerteeinrichtung (9) ausgebildet ist, ein Maß für die Kritikalität des Flugbetriebs basierend auf dem Messwert und dem Getriebeparameter zu bewerten.

9. Überwachungssystem (5) nach einem der Ansprüche 6 bis 8, **gekennzeichnet durch** eine Anzeigeeinrichtung (12), wobei die Anzeigeeinrichtung (12) ausgebildet ist, die Kritikalität des Flugbetriebs anzuzeigen.

10. Überwachungssystem (5) nach einem der Ansprüche 6 bis 9, **dadurch gekennzeichnet, dass** zwischen den beiden Kontakten (13, 14) ein v-förmiger Spalt gebildet ist, welcher dazu eingerichtet ist, mehrere der ferromagnetischen Partikel anzuhäufen.

11. Luftfahrzeug (1), **gekennzeichnet durch** ein Überwachungssystem (5) nach einem der Ansprüche 6 bis 10.

## Claims

1. A method of monitoring a gearbox (2) in an aircraft (1), wherein the gearbox (2) comprises at least one gearbox section (3) with at least one measuring area (M), wherein a first and a second electrical contact (13, 14) are arranged in the measuring area (M) at a distance (A) with respect to each other, in which:
- a magnetic field (F) is generated in the gearbox section (3), wherein the magnetic field (F) is configured to move at least one ferromagnetic particle (17) disposed in the gearbox section (2) towards the measuring area (M), so that a bridge (16) produced from one or more of the ferromagnetic particles (17) is formed between the two electrical contacts (13, 14);
- a measured value is generated on the basis of an electrical parameter between the two electrical contacts (13, 14);
- a measure of the criticality of a flight operation is determined by an evaluation module (10) on the basis of the generated measured value, wherein a warning signal is generated on the basis of the measure of the criticality and, based on the warning signal, the measure of the criticality of the flight operation is indicated, wherein the measure of the criticality is classified into at least two criticality levels (S1, S2, S3), and a respective warning signal is generated for each of the criticality levels (S1, S2, S3), and wherein no criticality is indicated in a first criticality level (S1), a low criticality is indicated in a second criticality level (S2) and a high criticality is indicated in a third criticality level (S3),
**characterized in that** a detected particle size and/or number of particles is determined for the generated measured value using an evaluation criterion stored in the evaluation module (10), wherein the measure of the criticality is classified into the first criticality level (S1) if the number of particles detected within a specified time period is equal to zero, wherein the measure of the criticality is classified into the second criticality level (S2) if the detected particle size is smaller than or equal to a specified maximum permissible particle size and the number of particles detected within the specified time period is equal to one, and wherein the measure of the criticality is classified into the third criticality level (S3) if the detected particle size is larger than the specified maximum permissible particle size and/or the number of particles detected within the specified time period is greater than one.

2. The method of claim 1, **characterized in that** the measure of the criticality is assessed on the basis of at least one further measured value which is detected independently of the generated measured value.

3. The method of claim 2, **characterized in that** a low criticality is indicated if the generated measured value and/or the further measured value are classified into the second criticality level (S2), and **in that** a high criticality is indicated if both the generated measured value and/or the further measured value are classified into the third criticality level (S3).

4. The method of claim 2 or claim 3, **characterized in that** the further measured value is output upon a detection of at least one further ferromagnetic particle (17) in a further measuring area (M) of the gearbox (2) and/or is generated upon a change in a gearbox parameter of the gearbox (2).

5. The method of one of the preceding claims, **characterized in that** the electrical parameter is an ohmic resistance and/or a voltage.

6. A monitoring system (5) for a gearbox (2) in an aircraft (1),
with at least one particle detection sensor (6) for the detection of ferromagnetic particles (17) in a measuring area (M),
- wherein the particle detection sensor (6) is arranged in a gearbox section (3) of the gearbox (2),
- wherein the particle detection sensor (6) comprises two electrical contacts (13, 14) which are distanced from each other,
- wherein the particle detection sensor (6) comprises a magnet (15) for generating a magnetic field (F), wherein the magnet (15) is configured to move a ferromagnetic particle disposed in the gearbox section (3) into the measuring area (M) of the particle detection sensor (6), so that an electrically conductive bridge (16) produced from one or more of the ferromagnetic particles (17) is formed between the two electrical contacts (13, 14),
- wherein the particle detection sensor (6) is configured to output a measured value on the basis of an electrical parameter between the two electrical contacts (13, 14),
with an evaluation device (9) for evaluating the measured value,
- wherein the evaluation device (9) is configured to determine a measure of the criticality of the flight operation by evaluating the measured value, wherein a warning signal is generated by the evaluation device (9) on the basis of the measure of the criticality and the measure of the criticality of the flight operation is indicated on the basis of the warning signal, wherein the measure of the criticality is classified into at least two criticality levels (S1, S2, S3) and a respective warning signal is generated for each of the criticality levels (S1 , S2, S3), and wherein no criticality is indicated in a first criticality level (S1), a low criticality is indicated in a second criticality level (S2) and a high criticality is indicated in a third criticality level (S3), **characterized in that** the evaluation device (9) comprises an evaluation module (10) in which an evaluation criterion is stored in order to determine a detected particle size and/or number of particles for the measured value using the evaluation criterion, wherein the measure of the criticality is classified into the first criticality level (S1) if the number of particles detected within a specified period of time is equal to zero, wherein the measure of the criticality is classified into the second criticality level (S2) if the detected particle size is less than or equal to a specified maximum permissible particle size and the number of particles detected within the specified period of time is equal to one, and wherein the measure of the criticality is classified into the third criticality level (S3) if the detected particle size is greater than the specified maximum permissible particle size and/or the number of particles detected within the specified period of time is greater than one.

7. The monitoring system (5) of claim 6, **characterized by** at least one further particle detection sensor (7) for detecting ferromagnetic particles (17) in a further measuring area (M) of the gearbox (2), wherein the further particle detection sensor (7) is arranged in the gearbox section (3) or a further gearbox section (4), wherein the further particle detection sensor (7) is configured to output a further measured value upon detecting at least one further ferromagnetic particle (17), wherein the evaluation device (9) is configured to assess a measure of the criticality of the flight operation on the basis of the measured value and the further measured value.

8. The monitoring system (5) of claim 6 or claim 7, **characterized by** at least one gearbox monitoring sensor (8) for detecting a gearbox parameter of the gearbox (2), wherein the gearbox monitoring sensor (8) is configured to output the gearbox parameter as a further measured value, wherein the evaluation device (9) is configured to assess a measure of the criticality of the flight operation on the basis of the measured value and the gearbox parameter.

9. The monitoring system (5) of one of claims 6 to 8, **characterized by** an indicator device (12), wherein the indicator device (12) is configured to indicate the criticality of the flight operation.

10. The monitoring system (5) of one of claims 6 to 9, **characterized in that** a V-shaped gap is formed between the two contacts (13, 14), the gap being configured to accumulate several of the ferromagnetic particles.

11. An aircraft (1) **characterized by** a monitoring system (5) according to one of claims 6 to 10.

## Revendications

1. Procédé de surveillance d'une boîte de vitesses (2) dans un aéronef (1), dans lequel la boîte de vitesses (2) comprend au moins une section de transmission (3) avec au moins une zone de mesure (M), un premier et un second contact électrique (13, 14) étant agencés dans la zone de mesure (M) à une distance (A) l'un de l'autre, dans lequel :
- un champ magnétique (F) est généré dans la section de transmission (3), dans lequel le champ magnétique (F) est conçu pour déplacer au moins une particule ferromagnétique (17) disposée dans la section de transmission (3) vers la zone de mesure (M), de telle sorte qu'un pont (16) d'une ou plusieurs des particules ferromagnétiques (17) se forme entre les deux contacts électriques (13, 14) ;
- une valeur de mesure est générée sur la base d'une valeur caractéristique électrique entre les deux contacts électriques (13, 14) ;
- sur la base de la valeur de mesure générée, une mesure de la criticité d'un fonctionnement en vol est déterminée par un module d'évaluation (10), dans lequel un signal d'avertissement est généré sur la base de la mesure de la criticité et la mesure de la criticité du fonctionnement en vol est affichée sur la base du signal d'avertissement, dans lequel la mesure de la criticité est divisée en au moins deux niveaux de criticité (S1, S2, S3) et un signal d'avertissement correspondant est généré pour chacun des niveaux de criticité (S1, S2, S3), et dans lequel aucune criticité n'est affichée dans un premier niveau de criticité (S1), une faible criticité est affichée dans un deuxième niveau de criticité (S2) et une haute criticité est affichée dans un troisième niveau de criticité (S3),
**caractérisé en ce qu'**en utilisant un critère d'évaluation stocké dans le module d'évaluation (10), une taille de particule et/ou un nombre de particules détectés sont déterminés pour la valeur de mesure générée, dans lequel la mesure de la criticité est classée dans le premier niveau de criticité (S1) si le nombre de particules détecté est égal à zéro pendant une période de temps définie, dans lequel la mesure de la criticité est classée dans le deuxième niveau de criticité (S2) si la taille de particule détectée est inférieure ou égale à une taille de particule maximale autorisée prédéterminée et si le nombre de particules détecté est égal à un pendant la période de temps définie, et la mesure de la criticité est classée dans le troisième niveau de criticité (S3) si la taille de particule détectée est supérieure à la taille de particule maximale autorisée prédéterminée et/ou si le nombre de particules détectée est supérieur à un pendant la période de temps définie.

2. Procédé selon la revendication 1, **caractérisé en ce que** la mesure de la criticité est évaluée sur la base d'au moins une valeur de mesure supplémentaire qui est enregistrée indépendamment de la valeur de mesure générée.

3. Procédé selon la revendication 2, **caractérisé en ce qu'**une faible criticité est affichée lorsque la valeur de mesure générée et/ou la valeur de mesure supplémentaire sont classées dans le deuxième niveau de criticité (S2), et **en ce qu'**une criticité élevée est affichée lorsque la valeur de mesure générée et/ou la valeur de mesure supplémentaire sont classées dans le troisième niveau de criticité (S3).

4. Procédé selon la revendication 2 ou 3, **caractérisé en ce que** la valeur de mesure supplémentaire est émise lors de la détection d'au moins une particule ferromagnétique supplémentaire (17) dans une zone de mesure supplémentaire (M) de la boîte de vitesses (2) et/ou est générée lors d'une modification d'un paramètre de transmission de la boîte de vitesses (2).

5. Procédé selon l'une quelconque des revendications précédentes, **caractérisé en ce que** la valeur caractéristique électrique est une résistance ohmique et/ou une tension.

6. Système de surveillance (5) pour une boîte de vitesses (2) dans un aéronef (1),
avec au moins un capteur de détection de particules (6) pour la détection de particules ferromagnétiques (17) dans une zone de mesure (M),
- dans lequel le capteur de détection de particules (6) est agencé dans une section de transmission (3) de la boîte de vitesses (2),
- dans lequel le capteur de détection de particules (6) comprend deux contacts électriques (13, 14) espacés l'un de l'autre,
- dans lequel le capteur de détection de particules (6) comprend un aimant (15) pour générer un champ magnétique (F), dans lequel l'aimant (15) est conçu pour déplacer une particule ferromagnétique disposée dans la section de transmission (3) jusque dans la zone de mesure (M) du capteur de détection de particules (6), de telle sorte qu'un pont électriquement conducteur (16) se forme d'une ou plusieurs des particules ferromagnétiques (17) entre les deux contacts électriques (13, 14),
- dans lequel le capteur de détection de particules (6) est conçu pour émettre une valeur de mesure sur la base d'une valeur caractéristique électrique entre les deux contacts électriques (13, 14),
avec un dispositif d'évaluation (9) pour évaluer la valeur de mesure,
- dans lequel le dispositif d'évaluation (9) est conçu pour déterminer une mesure de la criticité du fonctionnement en vol par une évaluation de la valeur de mesure, dans lequel un signal d'avertissement est généré par le dispositif d'évaluation (9) sur la base de la mesure de la criticité et la mesure de la criticité du fonctionnement en vol est affichée sur la base du signal d'avertissement, dans lequel la mesure de la criticité est classée dans au moins deux niveaux de criticité (S1, S2, S3) et un signal d'avertissement correspondant est généré pour chacun des niveaux de criticité (S1, S2, S3), et dans lequel aucune criticité n'est affichée dans un premier niveau de criticité (S1), une faible criticité est affichée dans un deuxième niveau de criticité (S2) et une haute criticité est affichée dans un troisième niveau de criticité (S3),
**caractérisé en ce que** le dispositif d'évaluation (9) comprend un module d'évaluation (10) dans lequel un critère d'évaluation est stocké afin de déterminer une taille de particule et/ou un nombre de particules détectés pour la valeur de mesure sur la base du critère d'évaluation, dans lequel la mesure de la criticité est classée dans le premier niveau de criticité (S1) si le nombre de particules détecté est égal à zéro pendant une période de temps définie, dans lequel la mesure de la criticité est classée dans le deuxième niveau de criticité (S2) si la taille de particule détectée est inférieure ou égale à une taille de particule maximale autorisée prédéterminée et si le nombre de particules détecté est égal à un pendant la période de temps définie, et la mesure de la criticité est classée dans le troisième niveau de criticité (S3) si la taille de particule détectée est supérieure à la taille de particule maximale autorisée prédéterminée et/ou si le nombre de particules détectée est supérieur à un pendant la période de temps définie.

7. Système de surveillance (5) selon la revendication 6, **caractérisé par** au moins un capteur de détection de particules supplémentaire (7) pour la détection de particules ferromagnétiques (17) dans une zone de mesure supplémentaire (M) de la boîte de vitesses (2), dans lequel le capteur de détection de particules supplémentaire (7) est agencé dans la section de transmission (3) ou dans une section de transmission supplémentaire (4), dans lequel le capteur de détection de particules supplémentaire (7) est conçu pour émettre une valeur de mesure supplémentaire lors de la détection d'au moins une particule ferromagnétique supplémentaire (17), dans lequel le dispositif d'évaluation (9) est conçu pour évaluer une mesure de la criticité du fonctionnement en vol sur la base de la valeur de mesure et de la valeur de mesure supplémentaire.

8. Système de surveillance (5) selon la revendication 6 ou 7, **caractérisé par** au moins un capteur de surveillance de transmission (8) pour détecter un paramètre de transmission de la boîte de vitesses (2), dans lequel le capteur de surveillance de transmission (8) est conçu pour émettre le paramètre de transmission en tant que valeur de mesure supplémentaire, dans lequel le dispositif d'évaluation (9) est conçu pour évaluer une mesure de la criticité du fonctionnement en vol sur la base de la valeur de mesure et du paramètre de transmission.

9. Système de surveillance (5) selon l'une quelconque des revendications 6 à 8, **caractérisé par** un dispositif d'affichage (12), dans lequel le dispositif d'affichage (12) est conçu pour afficher la criticité du fonctionnement en vol.

10. Système de surveillance (5) selon l'une quelconque des revendications 6 à 9, **caractérisé en ce qu'**un espace en forme de V est formé entre les deux contacts (13, 14), qui est agencé pour accumuler plusieurs des particules ferromagnétiques.

11. Aéronef (1), **caractérisé par** un système de surveillance (5) selon l'une quelconque des revendications 6 à 10.
